(19)

Europäisches
Patentamt
European
Patent Office
Office européen
des brevets

(11) **EP 4 040 353 B1**

(12) **EUROPEAN PATENT SPECIFICATION**

(45) Date of publication and mention
of the grant of the patent:
**26.07.2023 Bulletin 2023/30**

(21) Application number: **21820072.3**

(22) Date of filing: **02.06.2021**

(51) International Patent Classification (IPC):
$G06Q\ 10/04$ (2023.01)    $G06Q\ 10/08$ (2023.01)
$G06Q\ 50/26$ (2012.01)    $G06N\ 3/04$ (2023.01)
$G06N\ 3/08$ (2023.01)    $G06N\ 20/20$ (2019.01)
$H04W\ 4/90$ (2018.01)

(52) Cooperative Patent Classification (CPC):
**G06Q 10/04; G06N 3/045; G06N 3/08;
G06Q 10/08; G06Q 50/26; H04W 4/90**

(86) International application number:
**PCT/CN2021/097958**

(87) International publication number:
**WO 2022/134480 (30.06.2022 Gazette 2022/26)**

(54) **METHOD FOR ESTABLISHING RISK PREDICTION MODEL, REGIONAL RISK PREDICTION
METHOD AND CORRESPONDING APPARATUS**

VERFAHREN UND VORRICHTUNG ZUR ERSTELLUNG EINES RISIKOVORHERSAGEMODELLS
SOWIE VERFAHREN UND VORRICHTUNG ZUR VORHERSAGE REGIONALER RISIKEN

PROCÉDÉ D'ÉTABLISSEMENT D'UN MODÈLE DE PRÉDICTION DE RISQUE, PROCÉDÉ DE
PRÉDICTION DE RISQUE RÉGIONAL ET APPAREIL CORRESPONDANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2020 CN 202011515953**

(43) Date of publication of application:
**10.08.2022 Bulletin 2022/32**

(73) Proprietor: **Beijing Baidu Netcom
Science and Technology Co., Ltd.
Beijing 100085 (CN)**

(72) Inventors:
  • **HUANG, Jizhou
    Beijing 100085 (CN)**
  • **ZHOU, Jingbo
    Beijing 100085 (CN)**
  • **ZHOU, An
    Beijing 100085 (CN)**
  • **LIU, Ji
    Beijing 100085 (CN)**
  • **XIONG, Haoyi
    Beijing 100085 (CN)**

  • **DOU, Dejing
    Beijing 100085 (CN)**
  • **WANG, Haifeng
    Beijing 100085 (CN)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
**CN-A- 109 902 880    CN-A- 110 689 184
CN-A- 110 993 119    CN-A- 111 128 399
CN-A- 111 626 490    CN-A- 111 768 873
CN-A- 112 508 300    US-A1- 2017 242 972
US-A1- 2019 130 212**

Remarks:
The file contains technical information submitted after
the application was filed and not included in this
specification

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

## Description

The present application claims priority to Chinese Patent Application No. 2020115159533, filed on December 21, 2020, entitled "Method and Apparatus for Establishing Risk Prediction Model As Well As Regional Risk Prediction Method and Apparatus".

## TECHNICAL FIELD

The present disclosure relates to the field of computer application technologies, and particularly to a big data technology in the field of artificial intelligence technologies.

## BACKGROUND

A public emergency, such as epidemic spread, a biological disaster, a meteorological disaster, has a great influence on production, living and even safety of people. If a regional risk could be predicted timely and accurately, an emergency hazard might be effectively prevented from being spread, and targeted preventive measures may be taken, thus having a great significance.

There are some known predicting methods for traffic/pedestrian flow prediction known in the prior art.

CN 109 902 880 A provides a method for predicting urban crowd flow based on Seq2Seq to generate a confrontation network, which relates to the field of intelligent transportation, and is mainly used for urban pedestrian flow prediction, and plays an important role in urban traffic planning, citizen travel and reducing traffic risk.

CN 111 626 490 A provides a multi-task urban time-space prediction method based on adversarial learning, which relates to the field of intelligent transportation, is mainly used for urban pedestrian flow prediction, and plays an important role in urban transportation planning, citizen travel, and traffic risk reduction.

CN 110 689 184 A refers to a prediction of the flow of people at a traffic center, and in particular to a method for predicting the flow of people at a railroad crossing through deep learning.

These references all relate to traffic/pedestrian flow prediction. There is no efficient method for prediction of public emergency, such as epidemic spread, a biological disaster, a meteorological disaster.

## SUMMARY

The present disclosure provides a method and apparatus for establishing a risk prediction model as well as a regional risk prediction method and apparatus, so as to predict a regional risk.

According to a first aspect of the present disclosure, there is provided a method for establishing a risk prediction model with the features of claim 1, inter alia including:

> acquiring training data, the training data including a sample region set and annotation results of a risk grade of each sample region in the sample region set and a risk grade of a district to which each sample region belongs; and
> training an initial model including an encoder, a discriminator and a classifier using the training data, and obtaining the risk prediction model using the encoder and the classifier in the initial model after the training process;
> where the encoder performs a coding operation using region features of the sample regions to obtain a feature representation of each sample region; the discriminator identifies the risk grade of the district to which the sample region belongs according to the feature representation of the sample region; the classifier identifies the risk grade of the sample region according to the feature representation of the sample region; the initial model has training targets of minimizing a difference of identification of the sample regions belonging to the districts with different risk grades by the discriminator, and minimizing a difference between the identification result of the sample region by the classifier and the annotation result.

According to a second aspect of the present disclosure, there is provided a regional risk prediction method with the features of claim 4, inter alia including:

> extracting region features of a target block; and
> inputting the region features into a risk prediction model, and determining a risk grade of the target region according to a result output by the risk prediction model;
> where the risk prediction model is pre-established using the method as described above.

According to a third aspect of the present disclosure, there is provided an apparatus for establishing a risk prediction model with the features of claim 6, inter alia including:

a data acquiring unit configured to acquire training data, the training data including a sample region set and annotation results of a risk grade of each sample region in the sample region set and a risk grade of a district to which each sample region belongs; and

a model training unit configured to train an initial model including an encoder, a discriminator and a classifier using the training data, and obtain the risk prediction model using the encoder and the classifier in the initial model after the training process;

where the encoder performs a coding operation using region features of the sample regions to obtain a feature representation of each sample region; the discriminator identifies the risk grade of the district to which the sample region belongs according to the feature representation of the sample region; the classifier identifies the risk grade of the sample region according to the feature representation of the sample region; the initial model has training targets of minimizing a difference of identification of the sample regions belonging to the districts with different risk grades by the discriminator, and minimizing a difference between the identification result of the sample region by the classifier and the annotation result.

According to a fourth aspect of the present disclosure, there is provided a regional risk prediction apparatus with the features of claim 9, inter alia including:

a feature extracting unit configured to extract region features of a target block; and
a risk predicting unit configured to input the region features into a risk prediction model, and determine a risk grade of the target region according to a result output by the risk prediction model;
where the risk prediction model is pre-established by the apparatus as described above.

According to a fifth aspect of the present disclosure, there is provided an electronic device with the features of claim 11, inter alia including:

at least one processor; and
a memory connected with the at least one processor communicatively;
where the memory stores instructions executable by the at least one processor to enable the at least one processor to perform the method as mentioned above.

According to a sixth aspect of the present disclosure, there is provided a non-transitory computer readable storage medium including computer instructions, which, when executed by a computer, cause the computer to perform the method as mentioned above.

According to a seventh aspect of the present disclosure, there is provided a computer program product including a computer program which, when executed by a processor, cause the processor to perform the method as mentioned above.

From the above technical solution, the present disclosure provides the method for establishing a risk prediction model, and a risk prediction of the target region may be realized based on the established risk prediction model, thereby effectively preventing spread of an emergency hazard, and taking targeted preventive measures.

It should be understood that the statements in this section are not intended to identify key or critical features of the embodiments of the present disclosure, nor limit the scope of the present disclosure. Other features of the present disclosure will become apparent from the following description.

## BRIEF DESCRIPTION OF THE DRAWINGS

The drawings are used for better understanding the present solution and do not constitute a limitation of the present disclosure. In the drawings,

Fig. 1 is a flow chart of a method for establishing a risk prediction model according to an embodiment of the present disclosure;
Fig. 2 is a schematic structural diagram of a trained initial model according to an embodiment of the present disclosure;
Fig. 3 is a schematic structural diagram of the risk prediction model according to an embodiment of the present disclosure;
Fig. 4 is a regional risk prediction method according to an embodiment of the present disclosure;
Fig. 5 is a structural diagram of an apparatus for establishing a risk prediction model according to the present disclosure;
Fig. 6 is a structural diagram of a regional risk prediction apparatus according to the present disclosure; and
Fig. 7 is a block diagram of an electronic device configured to implement the embodiment of the present disclosure.

## DETAILED DESCRIPTION OF EMBODIMENTS

The following part will illustrate exemplary embodiments of the present disclosure with reference to the drawings, including various details of the embodiments of the present disclosure for a better understanding. The embodiments should be regarded only as exemplary ones. Therefore, those skilled in the art should appreciate that various changes or modifications can be made with respect to the embodiments described herein without departing from the scope and spirit of the present disclosure. Similarly, for clarity and conciseness, the descriptions of the known functions and structures are omitted in the descriptions below.

In an existing method for predicting a risk of a public emergency, such as an epidemic, a prediction is performed mainly by an infectious disease model using for example, a temporal and spatial distribution of infected users, a transmission speed of an infectious disease, a transmission path, or the like. However, such a model requires sufficient understanding and an accurate grasp of the epidemic as well as a sufficient professional knowledge background. However, spread of the epidemic is often sudden, and the onset of a disease is delayed (for example, there exists an incubation period, and a patient has no typical symptom in the incubation period), such that a risk prediction may have insufficient accuracy. In addition, such an infectious disease model is usually able to perform a prediction for a district where epidemic spread has occurred, but unable to perform a prediction for a district where the epidemic has not occurred.

In a method for establishing a risk prediction model according to the present disclosure, by learning features of regions in districts with different risk grades and features of the regions with different risk grades, a risk grade of a region with unknown risk conditions may be predicted based on the features. The method according to the present disclosure will be described below in detail in conjunction with an embodiment.

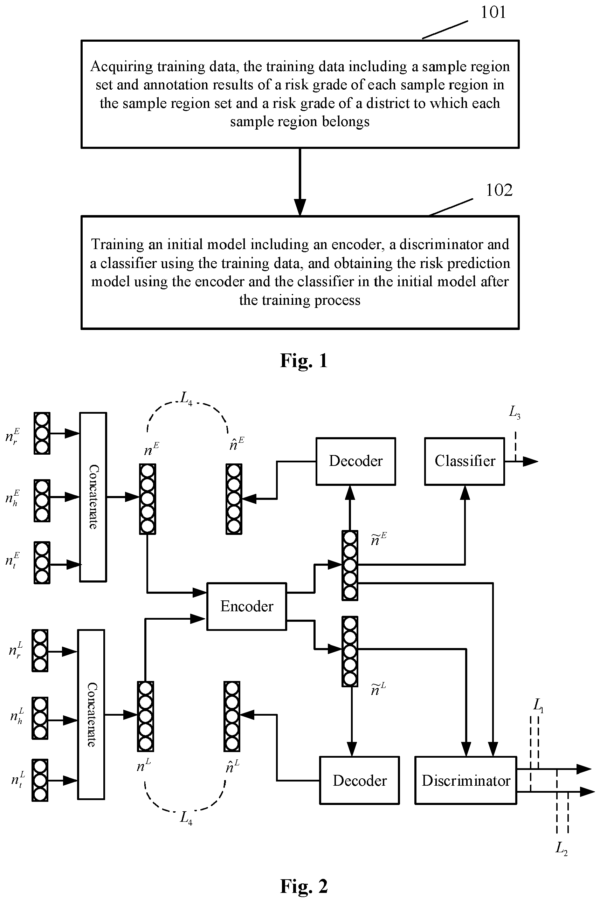

Fig. 1 is a flow chart of a method for establishing a risk prediction model according to an embodiment of the present disclosure, and as shown in Fig. 1, the method may include the following steps:

101: acquiring training data, the training data including a sample region set and annotation results of a risk grade of each sample region in the sample region set and a risk grade of a district to which each sample region belongs.

Regions with various risk grades in districts with various risk grades may be collected in advance as samples in the present disclosure. The district has a greater range than the region. For example, the district may be a province, a city, an administrative district, or the like. The region may be a block, a street, a school, a building, a factory, or the like.

The risk grade of the district may be divided into two types, such as a high risk grade and a low risk grade, and may also be divided into a plurality of types, such as a high risk grade, a medium risk grade, a low risk grade, a risk-free grade, or the like. The risk grade of the region may also be divided into two types, such as a high risk grade and a low risk grade, and may also be divided into a plurality of types, such as a high risk grade, a medium risk grade, a low risk grade, a risk-free grade, or the like. A specific division manner and specific division granularity are not limited in the present disclosure.

The risk grade of the district of each sample region and the risk grade of each sample region may be labeled in advance in the training data to be used in a subsequent model training process.

102: training an initial model including an encoder, a discriminator and a classifier using the training data, and obtaining the risk prediction model using the encoder and the classifier in the initial model after the training process.

The encoder performs a coding operation using region features extracted from the sample regions to obtain a feature representation of each sample region; the discriminator identifies the risk grade of the district to which the sample region belongs according to the feature representation of the sample region; the classifier identifies the risk grade of the sample region according to the feature representation of the sample region; the initial model has training targets of minimizing a difference of identification of the sample regions belonging to the districts with different risk grades by the discriminator, and minimizing a difference between the identification result of the sample region by the classifier and the annotation result.

From the above technical solution, the present disclosure provides the method for establishing a risk prediction model, and a risk prediction of the target region may be realized based on the established risk prediction model, thereby effectively preventing spread of an emergency hazard, and taking targeted preventive measures.

The steps in the above-mentioned embodiment are described in detail below with reference to an embodiment. In addition, since the method according to the present disclosure may be well applied to the epidemic risk prediction, the following embodiment will be described with the epidemic risk prediction as an example.

In the above-mentioned step 101, assuming that cities are divided into high risk cities and low risk cities in advance, some high risk blocks and some low risk blocks are selected from the known high risk cities and some low risk blocks are selected from the known low risk cities (usually, there are no high risk blocks in the low risk cities). A specific division manner is determined based on infection and spread conditions of the epidemic in the cities and the blocks. The sample region set is formed by the selected blocks, and the risk grades of the cities and the risk grades of the blocks are labeled for the blocks respectively, so as to constitute the training data.

Still further, the region features may be extracted separately for each block in the training data. The region feature extracted in the present disclosure may include at least one of a surrounding preset-type POI feature, a demographic feature, and a user travel feature. Unlike the existing infectious disease model, these region features employed in the present disclosure are not relevant to confirmed cases, and therefore, the prediction of a block risk may be performed in epidemic non-outbreak cities without prior experiences. The several features are described in detail below.

Surrounding preset-type POI feature:

living facilities around a block are usually related to a probability that the block is affected by an epidemic. For example, a block may be at a high risk due to a lack of basic living facilities, as residents may go farther to obtain living needs, and then, there exists a road infection possibility. Usually, the block lacking the basic living facilities lacks good management, also resulting in a high infection risk.

Based on the above considerations, the features of a preset type of POIs around a block may include, but are not limited to, the following two types:

The first type: information of a distance between the block and the nearest POI of the preset type. More than one type of POIs may be preset in the present disclosure, such as hospitals, clinics, schools, preschool educational institutions, bus stations, subway stations, airports, train stations, long-distance passenger stations, shopping malls, supermarkets, markets, shops, police offices, scenic spots, or the like. The features may be characterized by distances of the block from the nearest hospital, the nearest clinic, the nearest school, or the like.

The second type: a completeness degree of the living facilities in a preset distance range of the block. The completeness degree of the living facilities within, for example, 1 km may be adopted as one of the features in the present disclosure. That is, an evaluation may be performed based on conditions of hospitals, bus stations, supermarkets, shopping malls, markets, or the like, within 1 km. For example, 1 indicates a highest completeness degree, and 0 indicates a lowest completeness degree.

Demographic feature:

Since the epidemic is usually spread from person to person, the risk is required to be predicted in consideration of population density. Usually, the block with higher population density has a higher infection risk than the block with lower population density. Therefore, the population density may be taken as one of the demographic features.

In addition, different commuting distances also have a certain influence on the risk of the epidemic, and therefore, a distribution of the commuting distances of the block may be taken as one of the demographic features. As one implementation, an average commuting distance of the block may be used for characterization. The commuting distance may refer to a distance from a work place, a distance from a school, or the like.

Usually, different populations have different infection probabilities when exposed to the epidemic; for example, older and younger people are often more susceptible to infection due to weak immune systems. As another example, highly educated people have a higher degree of risk awareness and prevention, and therefore have a relatively lower infection probability. Based on this consideration, at least one of an age distribution, a gender distribution, an income distribution, a consumption ability distribution, an education level distribution, a marital status distribution, a life stage distribution, a job type distribution, an industry type distribution, or the like, may be selected as the demographic feature.

User travel feature:

Some related researches prove that user travel behaviors are usually closely related to epidemic spread. The user travel features involved in the present disclosure may include, but are not limited to, at least one of the following types:

The first type: a travel mode. For example, travel modes, such as walking, riding, public traffic, a private car, or the like, may be predefined.

The second type: a starting point-destination mode distribution. Information, such as a type of a destination, a distance between a starting point and the destination, or the like, may be included. The destinations may be classified into hospitals, restaurants, hotels, schools, or the like, in advance, a plurality of distance buckets are defined in advance, for example, 0 km-3 km, 3 km-10 km, 10 km-20 km, or the like, and the distance between the starting point and the destination is mapped to the corresponding distance bucket, which is taken as the feature.

The third type: a starting point-travel mode-destination mode distribution. The starting point refers to the current block, the travel mode and the destination type may be predefined, and then, top N combinations of counted combinations formed by the travel modes and the destination types of the block are used as the features. N is a preset positive integer, for example, 20.

It is observed that the above-mentioned features are relatively easy to obtain under any condition, and social and economic conditions as well as characteristics of spatial interaction activities of one region may be reflected at fine granularity of blocks, thereby realizing high-risk region identification at the fine granularity, and reducing a social cost.

The above-mentioned step 102 will be described below in detail in conjunction with an embodiment. First, a structure of the initial model used in the training process is described. As shown in Fig. 2, the initial model may include an encoder, a discriminator and a classifier, and may further include a decoder.

The region features extracted from the sample blocks are used as input of the encoder, and since the sample blocks belonging to the cities with different risk grades may be used in an actual training process, the sample blocks of the high risk cities and the sample blocks of the low risk cities are taken as examples in this embodiment. The surrounding preset-type POI feature, the demographic feature and the user travel feature of the sample block of the high risk city are represented by $n_r^E$, $n_h^E$ and $n_t^E$ respectively, and the surrounding preset-type POI feature, the demographic feature and the user travel feature of the sample block of the low risk city are represented by $n_r^L$, $n_h^L$ and $n_t^L$ respectively. $n_r^E$, $n_h^E$ and $n_t^E$ are fused, for example, are concatenated, to obtain the feature $n^E$ of the sample block of the high risk city. $n_r^L$, $n_h^L$ and $n_t^L$ are fused, for example, are concatenated to obtain the feature $n^L$ of the sample block of the low risk city.

$n^E$ is used as the input of the encoder and encoded by the encoder to obtain the feature representation $\tilde{n}^E$ of the sample block of the high risk city. Similarly, $n^L$ is used as the input of the encoder and encoded by the encoder to obtain the feature representation $\tilde{n}^L$ of the sample block of the low risk city. The encoder may be regarded to perform transformation on an input feature vector to obtain a new probability distribution.

In general, if experiences are wished to be learned from cities having massive outbreaks (i.e., high risk cities), these experiences are often required to have some commonality between different cities, and are not unique characteristics of the cities. How to learn these common features is a very important problem in the model training process. In the present disclosure, this problem is solved by training a discrimination model.

The discrimination model has functions of discriminating the risk grade of the city from which the feature representation originates according to the input $\tilde{n}^E$, and discriminating the risk grade of the city from which the feature representation originates according to the input $\tilde{n}^L$. The training process has an important training target of, after the coding operation of the encoder, enabling the obtained feature representation to make the discrimination model unable to distinguish the city from which the feature representation originates as far as possible, that is, minimizing a difference of identification by the discriminator of the sample regions belonging to the districts with different risk grades, which enables the encoder to learn the common features between the cities. From the training target, a loss function, referred to as a second loss function $L_2$, may be constructed, such as:

$$L_2 = -\left[\log\left(D\left(\tilde{n}^E\right)\right) + \log\left(D\left(\tilde{n}^L\right)\right)\right]$$

where $D(\ )$ represent an identification result of the discrimination model.

Further, besides learning the common features between the cities, the discrimination model is required to guarantee its own function (i.e., identification of the risk grade of the city from which the feature representation originates). Therefore, a loss function, referred to as a first loss function $L_1$, may be constructed in an adversarial learning manner, and the loss function is used for training the discrimination model to minimize the difference between the result of identification of the sample region by the discriminator and the annotation result. This loss function may be, for example,

$$L_1 = -\left[\log\left(D\left(\tilde{n}^E\right)\right) + \log\left(1 - D\left(\tilde{n}^L\right)\right)\right]$$

In the adversarial learning process, the discriminator continuously learns how to distinguish the risk grades of the cities from which $\tilde{n}^E$ and $\tilde{n}^L$ originate under an influence of $L_1$, which may result in an increase of $L_2$. Then, the encoder learns the common features as far as possible under the influence of $L_2$, so as to reduce $L_2$, such that the encoder and the discriminator perform continuous adversarial behaviors in the learning process, so as to finally reach a balance. At this point, the discriminator is unable to distinguish the sample blocks in the high risk cities and the low risk cities, and the encoder learns the common features between the sample blocks in the high risk cities and the sample blocks in the low risk cities.

Using the above-mentioned learning method, the common features between the sample blocks of the high risk cities and the sample blocks of the low risk cities may be learned, but the features of the sample blocks are not able to be learned to guide the identification of the risk grades of the blocks. Therefore, in the initial model, the risk grade of the block is identified by the classifier.

The classifier identifies the risk grade of the corresponding sample block according to $\tilde{n}^E$, with a training target of minimizing the difference between the result of the identification of the sample region by the classifier and the annotation result. In this regard, a loss function, i.e., a third loss function , $L_3$ may be constructed. This loss function may be, for example,

$$L_3 = - y^E \log\left(C(\tilde{n}^E)\right) - (1 - y^E) \log\left(1 - C(\tilde{n}^E)\right)$$

where $y^E$ represents the annotation result, and $C(\tilde{n}^E)$ represents the result of the identification of $\tilde{n}^E$ by the classifier.

The encoder and the classifier are optimized using the loss function, such that the encoder further learns the features capable of guiding the identification of the risk grade of the block on the basis of learning the common features between the cities. The classifier is guided to learn a capability of identifying the risk grade of the block. It should be additionally noted that the above-mentioned classifier is described with binary classification as an example, but a multi-classification classifier may be used in an actual model.

Still further, in order to enable the encoder to learn the features of the block as far as possible, an encoder-decoder framework is added in the present disclosure for feature reconstruction.

The encoder has a function of reconstructing the features of the region using the input feature representation of the sample block. That is, $n^E$ is reconstructed to obtain the vector representation $\hat{n}^E$ with a consistent dimension with $n^E$. $n^L$ is reconstructed to obtain the vector representation $\hat{n}^L$ with a consistent dimension with $n^L$. The encoder has an optimal target of recovering the original vector representation, that is, minimizing the difference between the reconstructed region features and the region features extracted from the sample region. Accordingly, a fourth loss function $L_4$ may be constructed. This loss function may be, for example,

$$L_4 = \left\| \hat{n}^E, n^E \right\|_2 + \left\| \hat{n}^L, n^L \right\|_2$$

The encoder and the decoder are optimized using $L_4$, such that the feature representation learned by the encoder still has the capability of describing the characteristics of one block.

In conclusion, it is observed that, as a preferred embodiment, in the process of training the initial model, the above-mentioned four loss functions are used to optimize and update the model parameters. Specifically, in each iteration process, parameters of the discriminator are optimized and updated using $L_1$, parameters of the encoder are optimized and updated using $L_2$, $L_3$ and $L_4$, and parameters of the classifier and the decoder are optimized and updated using $L_3$ and $L_4$.

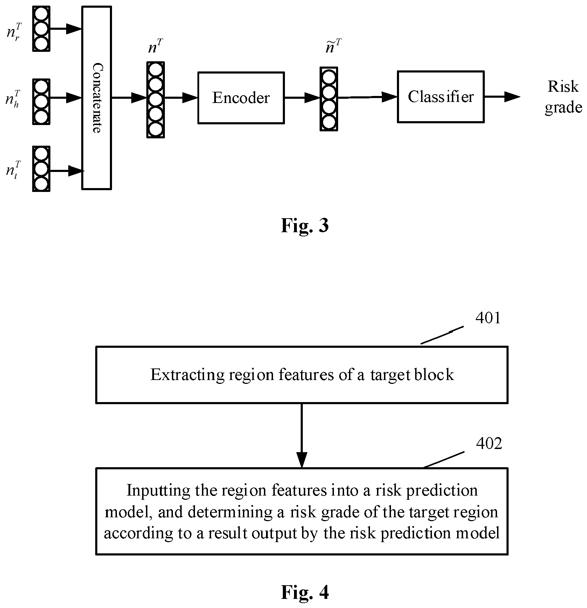

After the initial model is trained, for example, after the model converges or a preset iteration number is reached, the risk prediction model is obtained by the trained encoder and the trained classifier. That is, although the discriminator and the decoder are used in the training process to assist the training operation, only the encoder and the classifier are used in the actually obtained risk prediction model, which is shown in Fig. 3.

Fig. 4 is a regional risk prediction method according to an embodiment of the present disclosure, and the method is implemented based on the above-mentioned established risk prediction model. As shown in Fig. 4, the method includes: 401: extracting region features of a target block.

A manner of extracting the region features in the step is consistent with that of the region features adopted in the process of training the risk prediction model. The region feature may also include at least one of a surrounding preset-type POI feature, a demographic feature, and a user travel feature. For specific content of the region feature, reference is made to the related description in the embodiment shown in Fig. 1, which is not repeated herein.

402: inputting the region features into a risk prediction model, and determining a risk grade of the target region according to a result output by the risk prediction model.

As shown in Fig. 3, the surrounding preset-type POI feature, the demographic feature, and the user travel feature of the sample block of the high risk city are represented by $n_r^T$ , $n_h^T$ and $n_t^T$ respectively. $n_r^T$ , $n_h^T$ and $n_t^T$ are fused, for example, are concatenated to obtain the feature $n^T$ of the target block.

$n^T$ is used as the input of the encoder and encoded by the encoder to obtain the feature representation $\tilde{n}^T$ of the target block. The classifier identifies the risk grade of the corresponding sample block according to $\tilde{n}^T$.

It is observed that, in the above-mentioned process of predicting the risk grade of the target region, information of the district to which the target region belongs is not required, and the prediction of the risk grade is independent of the district.

As a typical application scenario, the present disclosure may be used to predict the risk grade of the region during epidemic spread. With the solution, potential high risk regions may be identified in districts without massive epidemic outbreaks, thereby having a great guiding significance for prevention and control of the epidemic.

The method according to the present disclosure is described above in detail, and an apparatus according to the present disclosure will be described below in detail in conjunction with an embodiment.

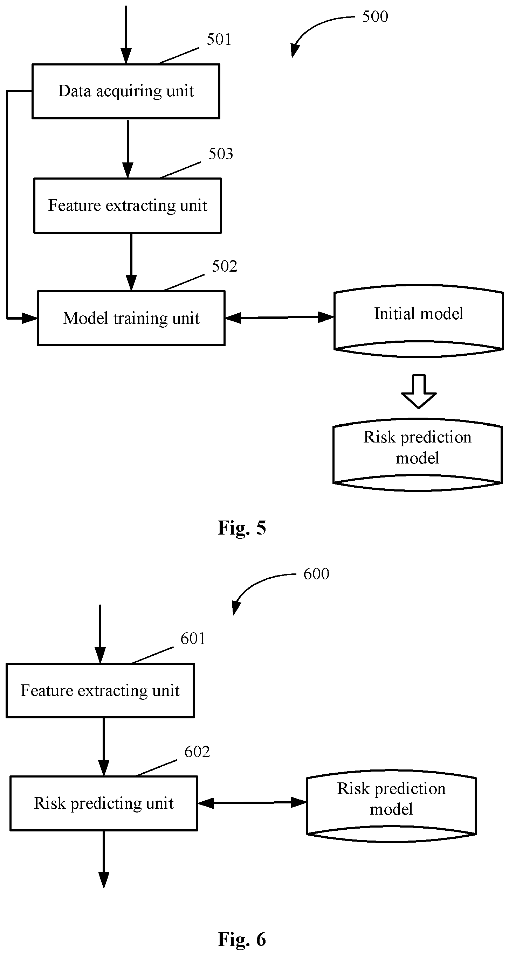

Fig. 5 is a structural diagram of an apparatus for establishing a risk prediction model according to the present disclosure; the apparatus may be configured as an application located at a server, or a functional unit, such as a plug-in or software development kit (SDK) located in the application of the server, or the like, or be located at a computer terminal with high computing power, which is not particularly limited in the embodiment of the present disclosure. As shown in Fig. 5, the apparatus 500 may include a data acquiring unit 501 and a model training unit 502, and may further include a feature extracting unit 503. The main functions of each constitutional unit are as follows.

The data acquiring unit 501 is configured to acquire training data, the training data including a sample region set and annotation results of a risk grade of each sample region in the sample region set and a risk grade of a district to which each sample region belongs.

The model training unit 502 is configured to train an initial model including an encoder, a discriminator and a classifier using the training data, and obtain the risk prediction model using the encoder and the classifier in the initial model after the training process.

The encoder performs a coding operation using region features of the sample regions to obtain a feature representation of each sample region; the discriminator identifies the risk grade of the district to which the sample region belongs according to the feature representation of the sample region; the classifier identifies the risk grade of the sample region according to the feature representation of the sample region; the initial model has training targets of minimizing a difference of identification of the sample regions belonging to the districts with different risk grades by the discriminator, and minimizing a difference between the identification result of the sample region by the classifier and the annotation result.

The feature extracting unit 503 is configured to acquire the region feature of the sample region, including at least one of: a surrounding preset-type POI feature, a demographic feature, and a user travel feature.

The surrounding preset-type POI feature includes at least one of information of a distance between the sample region and a nearest POI of a preset type, and a completeness degree of living facilities in a preset distance range of the sample region.

The demographic feature includes at least one of a population density condition, a commuting distance distribution, an age distribution, a gender distribution, an income distribution, a consumption ability distribution, an education level distribution, a marital status distribution, a life stage distribution, a job type distribution and an industry type distribution.

The user travel feature includes at least one of a travel mode, a starting point-destination mode distribution, and a starting point-travel mode-destination mode distribution.

As a preferred embodiment, the above-mentioned initial model may further include a decoder. The decoder reconstructs the region feature according to the feature representation of the sample region; the training process also has a target of minimizing a difference between the region feature reconstructed by the decoder and the region feature extracted from the sample region.

As a preferred embodiment, in the process of training the initial model, the model training unit 502 optimizes parameters of the discriminator using a first loss function, optimizes parameters of the encoder using a second loss function, a third loss function and a fourth loss function, optimizes parameters of the classifier using the third loss function, and optimizes parameters of the decoder using the fourth loss function.

The first loss function is used to minimize a difference between a result of identification of the sample region by the discriminator and the annotation result.

The second loss function is used to minimize the difference of the identification of the sample regions belonging to the districts with different risk grades by the discriminator.

The third loss function is used to minimize the difference between the result of the identification of the sample region by the classifier and the annotation result.

The fourth loss function is used to minimize the difference between the region feature reconstructed by the decoder and the region feature extracted from the sample region.

Fig. 6 is a structural diagram of a regional risk prediction apparatus according to the present disclosure; the apparatus may be configured as an application located at a server, or a functional unit, such as a plug-in or software development kit (SDK) located in the application of the server, or the like, or be located at a computer terminal with high computing power, which is not particularly limited in the embodiment of the present disclosure. As shown in Fig. 6, the apparatus 600 may include a feature extracting unit 601 and a risk predicting unit 602. The main functions of each constitutional unit are as follows.

The feature extracting unit 601 is configured to extract region features of a target block.

The risk predicting unit 602 is configured to input the region features into a risk prediction model, and determine a risk grade of the target region according to a result output by the risk prediction model.

The risk prediction model is pre-established by the apparatus shown in Fig. 5.

As a typical application scenario, the risk grade of the region predicted by the above-mentioned regional risk prediction apparatus is a risk grade of epidemic spread.

The embodiments in the present disclosure are described progressively, and mutual reference may be made to same and similar parts among the embodiments, and each embodiment focuses on differences from other embodiments. In particular, since the apparatus embodiment is substantially similar to the method embodiment, the description is relatively simple, and reference may be made to the corresponding description of the method embodiment for relevant points.

It should be noted here that the present disclosure may be applied to a typical application scenario, such as the risk grade prediction of epidemic spread, but besides this application scenario, the present disclosure may also be reasonably expanded within the scope of the idea of the present disclosure to be applied to other scenarios. The correspondingly extracted region features may be different when the present disclosure is applied to other application scenarios.

According to the embodiment of the present disclosure, there are also provided an electronic device, a readable storage medium and a computer program product.

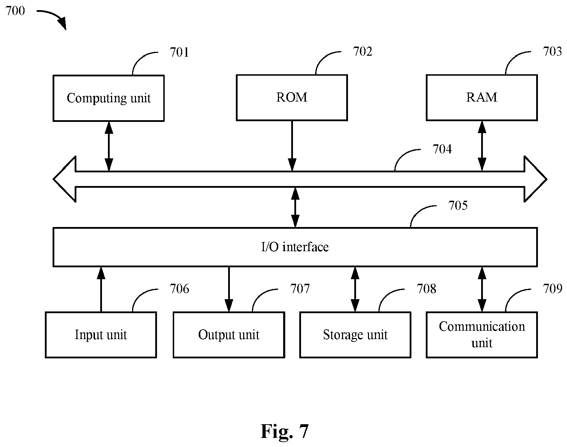

Fig. 7 is a block diagram of an electronic device configured to implement the embodiment of the present disclosure. The electronic device is intended to represent various forms of digital computers, such as laptop computers, desktop computers, workstations, personal digital assistants, servers, blade servers, mainframe computers, and other appropriate computers. The electronic device may also represent various forms of mobile apparatuses, such as personal digital processors, cellular telephones, smart phones, wearable devices, and other similar computing apparatuses. The components shown herein, their connections and relationships, and their functions, are meant to be exemplary only, and are not meant to limit implementation of the present disclosure described and/or claimed herein.

As shown in Fig. 7, the device 700 includes a computing unit 701 which may perform various appropriate actions and processing operations according to a computer program stored in a read only memory (ROM) 702 or a computer program loaded from a storage unit 708 into a random access memory (RAM) 703. Various programs and data necessary for the operation of the device 700 may be also stored in the RAM 703. The computing unit 701, the ROM 702, and the RAM 703 are connected with one other through a bus 704. An input/output (I/O) interface 705 is also connected to the bus 704.

The plural components in the device 700 are connected to the I/O interface 705, and include: an input unit 706, such as a keyboard, a mouse, or the like; an output unit 707, such as various types of displays, speakers, or the like; the storage unit 708, such as a magnetic disk, an optical disk, or the like; and a communication unit 709, such as a network card, a modem, a wireless communication transceiver, or the like. The communication unit 709 allows the device 700 to exchange information/data with other devices through a computer network, such as the Internet, and/or various telecommunication networks.

The computing unit 701 may be a variety of general and/or special purpose processing components with processing and computing capabilities. Some examples of the computing unit 701 include, but are not limited to, a central processing unit (CPU), a graphic processing unit (GPU), various dedicated artificial intelligence (AI) computing chips, various computing units running machine learning model algorithms, a digital signal processor (DSP), and any suitable processor, controller, microcontroller, or the like. The computing unit 701 performs the methods and processing operations described above, such as the method for establishing a risk prediction model or the regional risk prediction method. For example, in some embodiments, the method for establishing a risk prediction model or the regional risk prediction method may be implemented as a computer software program tangibly contained in a machine readable medium, such as the storage unit 708.

In some embodiments, part or all of the computer program may be loaded and/or installed into the device 700 via the ROM 502 and/or the communication unit 709. When the computer program is loaded into the RAM 703 and executed by the computing unit 701, one or more steps of the method for establishing a risk prediction model and the regional risk prediction method described above may be performed. Alternatively, in other embodiments, the computing unit 701 may be configured to perform the method for establishing a risk prediction model or the regional risk prediction method by any other suitable means (for example, by means of firmware).

Various implementations of the systems and technologies described herein may be implemented in digital electronic circuitry, integrated circuitry, field programmable gate arrays (FPGA), application specific integrated circuits (ASIC), application specific standard products (ASSP), systems on chips (SOC), complex programmable logic devices (CPLD), computer hardware, firmware, software, and/or combinations thereof. The systems and technologies may be implemented in one or more computer programs which are executable and/or interpretable on a programmable system including at least one programmable processor, and the programmable processor may be special or general, and may receive data and instructions from, and transmit data and instructions to, a storage system, at least one input apparatus, and at least one output apparatus.

Program codes for implementing the method according to the present disclosure may be written in any combination of one or more programming languages. These program codes may be provided to a processor or a controller of a general purpose computer, a special purpose computer, or other programmable data processing apparatuses, such that the program code, when executed by the processor or the controller, causes functions/operations specified in the flowchart and/or the block diagram to be implemented. The program code may be executed entirely on a machine, partly on a machine, partly on a machine as a stand-alone software package and partly on a remote machine, or entirely on a remote machine or a server.

In the context of the present disclosure, the machine readable medium may be a tangible medium which may contain or store a program for use by or in connection with an instruction execution system, apparatus, or device. The machine readable medium may be a machine readable signal medium or a machine readable storage medium. The machine readable medium may include, but is not limited to, an electronic, magnetic, optical, electromagnetic, infrared, or semiconductor system, apparatus, or device, or any suitable combination of the foregoing. More specific examples of the machine readable storage medium may include an electrical connection based on one or more wires, a portable computer disk, a hard disk, a random access memory (RAM), a read only memory (ROM), an erasable programmable read only memory (EPROM or flash memory), an optical fiber, a portable compact disc read only memory (CD-ROM), an optical storage device, a magnetic storage device, or any suitable combination of the foregoing.

To provide interaction with a user, the systems and technologies described here may be implemented on a computer having: a display apparatus (for example, a cathode ray tube (CRT) or liquid crystal display (LCD) monitor) for displaying information to a user; and a keyboard and a pointing apparatus (for example, a mouse or a trackball) by which a user may provide input for the computer. Other kinds of apparatuses may also be used to provide interaction with a user; for example, feedback provided for a user may be any form of sensory feedback (for example, visual feedback, auditory feedback, or tactile feedback); and input from a user may be received in any form (including acoustic, speech or tactile input).

The systems and technologies described here may be implemented in a computing system (for example, as a data server) which includes a back-end component, or a computing system (for example, an application server) which includes a middleware component, or a computing system (for example, a user computer having a graphical user interface or a web browser through which a user may interact with an implementation of the systems and technologies described here) which includes a front-end component, or a computing system which includes any combination of such back-end, middleware, or front-end components. The components of the system may be interconnected through any form or medium of digital data communication (for example, a communication network). Examples of the communication network include: a local area network (LAN), a wide area network (WAN) and the Internet.

A computer system may include a client and a server. Generally, the client and the server are remote from each other and interact through the communication network. The relationship between the client and the server is generated by virtue of computer programs which run on respective computers and have a client-server relationship to each other.

It should be understood that various forms of the flows shown above may be used and reordered, and steps may be added or deleted. For example, the steps described in the present application may be executed in parallel, sequentially, or in different orders, which is not limited herein as long as the desired results of the technical solution disclosed in the present disclosure may be achieved.

The above-mentioned implementations are not intended to limit the scope of the present disclosure. It should be understood by those skilled in the art that various modifications, combinations, sub-combinations and substitutions may be made, depending on design requirements and other factors.

**Claims**

1. A method for establishing a risk prediction model, comprising:

   acquiring (101) training data, the training data comprising a sample region set and annotation results of a risk grade of each sample region in the sample region set and a risk grade of a district to which each sample region belongs; and

   training (102) an initial model comprising an encoder, a discriminator and a classifier using the training data, and obtaining the risk prediction model using the encoder and the classifier in the initial model after the training process;

   wherein the encoder performs a coding operation using region features of the sample regions to obtain a feature representation of each sample region;

   the method being **characterized in that**

   the discriminator identifies the risk grade of the district to which the sample region belongs according to the feature representation of the sample region; the classifier identifies the risk grade of the sample region according to the feature representation of the sample region; the initial model has training targets of minimizing a difference of identification of the sample regions belonging to the districts with different risk grades by the discriminator, and minimizing a difference between the identification result of the sample region by the classifier and the annotation result,

wherein the region feature of the sample region comprises at least one of:

a surrounding preset-type POI feature, a demographic feature, and a user travel feature, and wherein the surrounding preset-type POI feature comprises at least one of: information of a distance between the sample region and a nearest POI of a preset type, and a completeness degree of living facilities in a preset distance range of the sample region;

the demographic feature comprises at least one of: a population density condition, a commuting distance distribution, an age distribution, a gender distribution, an income distribution, a consumption ability distribution, an education level distribution, a marital status distribution, a life stage distribution, a job type distribution and an industry type distribution;

the user travel feature comprises at least one of: a travel mode, a starting point-destination mode distribution, and a starting point-travel mode-destination mode distribution.

2. The method according to claim 1, wherein the initial model further comprises a decoder;

the decoder reconstructs the region feature according to the feature representation of the sample region; the training process also has a target of minimizing a difference between the region feature reconstructed by the decoder and the region feature extracted from the sample region.

3. The method according to claim 2, wherein in the process of training the initial model, parameters of the discriminator are optimized using a first loss function, parameters of the encoder are optimized using a second loss function, a third loss function and a fourth loss function, parameters of the classifier are optimized using the third loss function, and parameters of the decoder are optimized using the fourth loss function;

the first loss function is used to minimize a difference between a result of identification of the sample region by the discriminator and the annotation result;
the second loss function is used to minimize the difference of the identification of the sample regions belonging to the districts with different risk grades by the discriminator;
the third loss function is used to minimize the difference between the result of the identification of the sample region by the classifier and the annotation result;
the fourth loss function is used to minimize the difference between the region feature reconstructed by the decoder and the region feature extracted from the sample region.

4. A regional risk prediction method, **characterized by** comprising:

extracting (401) region features of a target block; and
inputting (402) the region features into a risk prediction model, and determining a risk grade of the target region according to a result output by the risk prediction model;
wherein the risk prediction model is pre-established using the method according to any one of claims of 1 to 3.

5. The method according to claim 4, wherein the risk grade is a risk grade of epidemic spread.

6. An apparatus for establishing a risk prediction model, comprising:

a data acquiring unit (501) configured to acquire (101) training data, the training data comprising a sample region set and annotation results of a risk grade of each sample region in the sample region set and a risk grade of a district to which each sample region belongs; and
a model training unit (502) configured to train (102) an initial model comprising an encoder, a discriminator and a classifier using the training data, and obtain the risk prediction model using the encoder and the classifier in the initial model after the training process;
wherein the encoder performs a coding operation using region features of the sample regions to obtain a feature representation of each sample region; the discriminator identifies the risk grade of the district to which the sample region belongs according to the feature representation of the sample region; the classifier identifies the risk grade of the sample region according to the feature representation of the sample region; the initial model has training targets of minimizing a difference of identification of the sample regions belonging to the districts with different risk grades by the discriminator, and minimizing a difference between the identification result of the sample region by the classifier and the annotation result, wherein the apparatus further comprises:

a feature extracting unit (503) configured to acquire the region feature of the sample region, comprising at least one of: a surrounding preset-type POI feature, a demographic feature, and a user travel feature, and wherein the surrounding preset-type POI feature comprises at least one of information of a distance between the sample region and a nearest POI of a preset type, and a completeness degree of living facilities in a preset distance range of the sample region;

the demographic feature comprises at least one of: a population density condition, a commuting distance distribution, an age distribution, a gender distribution, an income distribution, a consumption ability distribution, an education level distribution, a marital status distribution, a life stage distribution, a job type distribution and an industry type distribution;

the user travel feature comprises at least one of: a travel mode, a starting point-destination mode distribution, and a starting point-travel mode-destination mode distribution.

7. The apparatus according to claim 6, wherein the initial model further comprises a decoder;

the decoder reconstructs the region feature according to the feature representation of the sample region;

the training process also has a target of minimizing a difference between the region feature reconstructed by the decoder and the region feature extracted from the sample region.

8. The apparatus according to claim 7, wherein in the process of training the initial model, the model training unit optimizes parameters of the discriminator using a first loss function, optimizes parameters of the encoder using a second loss function, a third loss function and a fourth loss function, optimizes parameters of the classifier using the third loss function, and optimizes parameters of the decoder using the fourth loss function;

the first loss function is used to minimize a difference between a result of identification of the sample region by the discriminator and the annotation result;

the second loss function is used to minimize the difference of the identification of the sample regions belonging to the districts with different risk grades by the discriminator;

the third loss function is used to minimize the difference between the result of the identification of the sample region by the classifier and the annotation result;

the fourth loss function is used to minimize the difference between the region feature reconstructed by the decoder and the region feature extracted from the sample region.

9. A regional risk prediction apparatus, **characterized by** comprising:

a feature extracting unit (601) configured to extract region features of a target block; and

a risk predicting unit (602) configured to input the region features into a risk prediction model, and determine a risk grade of the target region according to a result output by the risk prediction model;

wherein the risk prediction model is pre-established by the apparatus according to any one of claims of 6 to 8.

10. The apparatus according to claim 9, wherein the risk grade is a risk grade of epidemic spread.

11. An electronic device, comprising:

at least one processor; and

a memory connected with the at least one processor communicatively;

wherein the memory stores instructions executable by the at least one processor to cause the at least one processor to perform the method according to any one of claims 1 to 5.

12. A non-transitory computer readable storage medium comprising computer instructions, which, when executed by a computer, cause the computer to perform the method according to any one of claims 1 to 5.

13. A computer program product comprising a computer program which, when executed by a processor, cause the processor to perform the method according to any one of claims 1 to 5.

**Patentansprüche**

1. Verfahren zur Erstellung eines Risikovorhersagemodells, das umfasst:

   Erfassen (101) von Trainingsdaten, wobei die Trainingsdaten einen Probenbereichssatz und Annotationsergebnisse eines Risikograds eines Probenbereichs in dem Probenbereichssatz und eines Risikograds eines Gebiets, zu dem jeder Probenbereich gehört, umfassen; und
   Trainieren (102) eines Ausgangsmodells, das einen Encoder, einen Diskriminator und einen Klassifikator aufweist, mittels der Trainingsdaten, und Erhalten des Risikovorhersagemodells unter Verwendung des Encoders und des Klassifikators in dem Ausgangsmodell nach dem Trainingsprozess;
   wobei der Encoder einen Codiervorgang unter Verwendung der Bereichsmerkmale der Probenbereiche durchführt, um eine Merkmalsdarstellung jedes Probenbereichs zu erhalten;
   wobei das Verfahren **dadurch gekennzeichnet ist, dass**
   der Diskriminator den Risikograd des Gebiets, zu dem der Probenbereich gehört, gemäß der Merkmalsdarstellung des Probenbereichs identifiziert;
   der Klassifikator den Risikograd des Probenbereichs gemäß der Merkmalsdarstellung des Probenbereichs identifiziert;
   das Ausgangsmodell zum Trainingsziel hat, eine Identifikationsdifferenz der Probenbereiche, die zu den Gebieten mit verschiedenen Risikograden gehören, mittels des Diskriminators zu minimieren und eine Differenz zwischen dem Identifikationsergebnis des Probenbereichs mittels des Klassifikators und dem Annotationsergebnis zu minimieren,
   wobei das Bereichsmerkmal des Probenbereichs zumindest eines aus Folgendem aufweist:

      ein umgebendes POI-Merkmal vom voreingestellten Typ, ein demographisches Merkmal, und ein Benutzerreisemerkmal, und
      wobei das umgebende POI-Merkmal vom voreingestellten Typ zumindest eines aus Folgendem umfasst: Informationen zu einer Entfernung zwischen dem Probenbereich und einem nächsten POI eines voreingestellten Typs, und einen Vollständigkeitsgrad von Wohnanlagen in einem voreingestellten Entfernungsbereichs des Probenbereichs;
      wobei das demographische Merkmal zumindest eines aus Folgendem umfasst: einen Bevölkerungsdichtezustand, eine Pendelentfernungsverteilung, eine Altersverteilung, eine Geschlechterverteilung, eine Einkommensverteilung, eine Konsumfähigkeitsverteilung, eine Bildungsniveauverteilung, eine Familienstandverteilung, eine Lebensphasenverteilung, eine Jobtypverteilung und eine Industrietypverteilung;
      wobei das Benutzerreisemerkmal zumindest eines aus Folgendem umfasst: einen Reisemodus, eine Startpunkt-Zielmodus-Verteilung und eine Startpunkt-Reisemodus-Zielmodus-Verteilung.

2. Verfahren nach Anspruch 1, wobei das Ausgangsmodell ferner einen Decoder aufweist;

   wobei der Decoder das Bereichsmerkmal gemäß der Merkmalsdarstellung des Probenbereichs rekonstruiert;
   wobei der Trainingsprozess ferner zum Ziel hat, eine Differenz zwischen dem von dem Decoder rekonstruierten Bereichsmerkmals und dem aus dem Probenbereich extrahierten Bereichsmerkmal zu minimieren.

3. Verfahren nach Anspruch 2, wobei in dem Prozess des Trainierens des Ausgangsmodells Parameter des Diskriminators mittels einer ersten Verlustfunktion optimiert werden, Parameter des Encoders mittels einer zweiten Verlustfunktion, einer dritten Verlustfunktion und einer vierten Verlustfunktion optimiert werden, Parameter des Klassifikators mittels der dritten Verlustfunktion optimiert werden, und Parameter des Decoders mittels der vierten Verlustfunktion optimiert werden;

   wobei die erste Verlustfunktion dazu verwendet wird, eine Differenz zwischen einem Identifikationsergebnis des Probenbereichs mittels des Diskriminators und dem Annotationsergebnis zu minimieren;
   wobei die zweite Verlustfunktion dazu verwendet wird, die Identifikationsdifferenz der Probenbereiche, die zu den Gebieten mit verschiedenen Risikograden gehören, mittels des Diskriminators zu minimieren;
   wobei die dritte Verlustfunktion dazu verwendet wird, die Differenz zwischen dem Identifikationsergebnis des Probenbereichs mittels des Klassifikators und dem Annotationsergebnis zu minimieren;
   wobei die vierte Verlustfunktion dazu verwendet wird, die Differenz zwischen den von dem Decoder rekonstruierten Bereichsmerkmal und dem aus dem Probenbereich extrahierten Bereichsmerkmals zu minimieren.

4. Regionales Risikovorhersageverfahren, **dadurch gekennzeichnet, dass** das Verfahren umfasst:

Extrahieren (401) von Bereichsmerkmalen eines Zielblocks; und

Eingeben (402) der Bereichsmerkmale in ein Risikovorhersagemodell, und Bestimmen eines Risikograds des Zielbereichs gemäß einem von dem Risikovorhersagemodell ausgegebenen Ergebnis;

wobei das Risikovorhersagemodell mittels des Verfahrens nach einem der Ansprüche 1 bis 3 vorab erstellt wird.

5. Verfahren nach Anspruch 4, wobei der Risikograd ein Risikograd epidemischer Ausbreitung ist.

6. Vorrichtung zur Erstellung eines Risikovorhersagemodells, die aufweist:

eine Datenerfassungseinheit (501), die zum Erfassen (101) von Trainingsdaten ausgebildet ist, wobei die Trainingsdaten einen Probenbereichssatz und Annotationsergebnisse eines Risikograds jedes Probenbereichs in dem Probenbereichssatz und eines Risikograds eines Gebiets, zu dem jeder Probenbereich gehört, umfassen; und

eine Modelltrainingseinheit (502), die zum Trainieren (102) eines Ausgangsmodells, das einen Encoder, einen Diskriminator und einen Klassifikator aufweist, mittels der Trainingsdaten und zum Erhalten des Risikovorhersagemodells unter Verwendung des Encoders und des Klassifikators in dem Ausgangsmodell nach dem Trainingsprozess ausgebildet ist;

wobei der Encoder einen Codiervorgang unter Verwendung der Bereichsmerkmale der Probenbereiche durchführt, um eine Merkmalsdarstellung jedes Probenbereichs zu erhalten;

wobei der Diskriminator den Risikograd des Gebiets, zu dem der Probenbereich gehört, gemäß der Merkmalsdarstellung des Probenbereichs identifiziert;

wobei der Klassifikator den Risikograd des Probenbereichs gemäß der Merkmalsdarstellung des Probenbereichs identifiziert;

wobei das Ausgangsmodell zum Trainingsziel hat, eine Identifikationsdifferenz der Probenbereiche, die zu den Gebieten mit verschiedenen Risikograden gehören, mittels des Diskriminators zu minimieren und eine Differenz zwischen dem Identifikationsergebnis des Probenbereichs mittels des Klassifikators und dem Annotationsergebnis zu minimieren, wobei die Vorrichtung ferner aufweist:

eine Merkmalsextraktionseinheit (503), die zum Erfassen des Bereichsmerkmals des Probenbereichs ausgebildet ist, das zumindest eines aus Folgendem aufweist: ein umgebendes POI-Merkmal vom voreingestellten Typ, ein demographisches Merkmal, und ein Benutzerreisemerkmal, und wobei das umgebende POI-Merkmal vom voreingestellten Typ zumindest eines aus Informationen zu einer Entfernung zwischen dem Probenbereich und einem nächsten POI eines voreingestellten Typs und einen Vollständigkeitsgrad von Wohnanlagen in einem voreingestellten Entfernungsbereichs des Probenbereichs umfasst;

wobei das demographische Merkmal zumindest eines aus Folgendem umfasst: einen Bevölkerungsdichtezustand, eine Pendelentfernungsverteilung, eine Altersverteilung, eine Geschlechterverteilung, eine Einkommensverteilung, eine Konsumfähigkeitsverteilung, eine Bildungsniveauverteilung, eine Familienstandverteilung, eine Lebensphasenverteilung, eine Jobtypverteilung und eine Industrietypverteilung;

wobei das Benutzerreisemerkmal zumindest eines aus Folgendem umfasst: einen Reisemodus, eine Startpunkt-Zielmodus-Verteilung und eine Startpunkt-Reisemodus-Zielmodus-Verteilung.

7. Vorrichtung nach Anspruch 6, wobei das Ausgangsmodell ferner einen Decoder aufweist;

wobei der Decoder das Bereichsmerkmal gemäß der Merkmalsdarstellung des Probenbereichs rekonstruiert;

wobei der Trainingsprozess ferner zum Ziel hat, eine Differenz zwischen dem von dem Decoder rekonstruierten Bereichsmerkmal und dem aus dem Probenbereich extrahierten Bereichsmerkmal zu minimieren.

8. Vorrichtung nach Anspruch 7, wobei in dem Prozess des Trainierens des Ausgangsmodells die Modelltrainingseinheit Parameter des Diskriminators mittels einer ersten Verlustfunktion optimiert, Parameter des Encoders mittels einer zweiten Verlustfunktion, einer dritten Verlustfunktion und einer vierten Verlustfunktion optimiert, Parameter des Klassifikators mittels der dritten Verlustfunktion optimiert, und Parameter des Decoders mittels der vierten Verlustfunktion optimiert;

wobei die erste Verlustfunktion dazu verwendet wird, eine Differenz zwischen einem Identifikationsergebnis des Probenbereichs mittels des Diskriminators und dem Annotationsergebnis zu minimieren;

wobei die zweite Verlustfunktion dazu verwendet wird, die Identifikationsdifferenz der Probenbereiche, die zu den Gebieten mit verschiedenen Risikograden gehören, mittels des Diskriminators zu minimieren;

wobei die dritte Verlustfunktion dazu verwendet wird, die Differenz zwischen dem Ergebnis der Identifikation des Probenbereichs mittels des Klassifikators und dem Annotationsergebnis zu minimieren;
wobei die vierte Verlustfunktion dazu verwendet wird, die Differenz zwischen dem von dem Decoder rekonstruierten Bereichsmerkmal und dem aus dem Probenbereich extrahierten Bereichsmerkmal zu minimieren.

9. Regionale Risikovorhersagevorrichtung, **dadurch gekennzeichnet, dass** die Vorrichtung aufweist:

   eine Merkmalsextraktionseinheit (601), die zum Extrahieren der Bereichsmerkmale eines Zielblocks ausgebildet ist; und
   eine Risikovorhersageeinheit (602), die zum Eingeben der Bereichsmerkmale in ein Risikovorhersagemodell und zum Bestimmen eines Risikograds des Zielbereichs gemäß einem von dem Risikovorhersagemodell ausgegebenen Ergebnis ausgebildet ist;
   wobei das Risikovorhersagemodell mittels der Vorrichtung nach einem der Ansprüche 6 bis 8 vorab erstellt wird.

10. Vorrichtung nach Anspruch 9, wobei der Risikograd ein Risikograd epidemischer Ausbreitung ist.

11. Elektronische Vorrichtung, die aufweist:

   mindestens einen Prozessor; und
   einen Speicher, der mit dem mindestens einen Prozessor kommunikativ verbunden ist;
   wobei der Speicher Anweisungen speichert, die von dem mindestens einen Prozessor ausgeführt werden können, um zu bewirken, dass der mindestens eine Prozessor das Verfahren nach einem der Ansprüche 1 bis 5 durchführt.

12. Nichtflüchtiges computerlesbares Speichermedium, das Computeranweisungen aufweist, die bei Ausführung durch einen Computer bewirken, dass der Computer das Verfahren nach einem der Ansprüche 1 bis 5 durchführt.

13. Computerprogrammprodukt, das ein Computerprogramm aufweist, das bei Ausführung durch einen Prozessor bewirkt, dass der Prozessor das Verfahren nach einem der Ansprüche 1 bis 5 durchführt.

**Revendications**

1. Procédé permettant d'établir un modèle de prédiction de risque, comprenant :

   l'acquisition (101) de données d'entraînement, les données d'entraînement comprenant un ensemble de régions échantillons et des résultats d'annotation d'un niveau de risque de chaque région échantillon dans l'ensemble de régions échantillons et d'un niveau de risque d'un district auquel appartient chaque région échantillon ; et
   l'entraînement (102) d'un modèle initial comprenant un encodeur, un discriminateur et un classificateur à l'aide des données d'entraînement, et l'obtention du modèle de prédiction de risque à l'aide de l'encodeur et du classificateur dans le modèle initial après le processus d'entraînement ;
   dans lequel l'encodeur met en oeuvre une opération de codage à l'aide de caractéristiques de région des régions échantillons pour obtenir une représentation de caractéristiques de chaque région échantillon ;
   le procédé étant **caractérisé en ce que**
   le discriminateur identifie le niveau de risque du district auquel appartient la région échantillon selon la représentation de caractéristiques de la région échantillon ; le classificateur identifie le niveau de risque de la région échantillon selon la représentation de caractéristiques de la région échantillon ; le modèle initial a des objectifs d'entraînement consistant à minimiser une différence d'identification des régions échantillons appartenant aux districts avec différents niveaux de risque par le discriminateur, et à minimiser une différence entre le résultat d'identification de la région échantillon par le classificateur et le résultat d'annotation,
   dans lequel la caractéristique de région de la région échantillon comprend au moins l'une parmi :

   une caractéristique de POI de type prédéfini environnant, une caractéristique démographique et une caractéristique de déplacement d'utilisateur, et
   dans lequel la caractéristique de POI de type prédéfini environnant comprend au moins l'un parmi : des informations de distance entre la région échantillon et un POI le plus proche d'un type prédéfini, et un degré d'exhaustivité d'installations d'habitation dans une plage de distance prédéfinie de la région échantillon ;
   la caractéristique démographique comprend au moins l'une parmi : une condition de densité de population, une distribution de distance de navettage, une distribution d'âge, une distribution de sexe, une distribution de revenu, une distribution de capacité de consommation, une distribution de niveau d'éducation, une distribution d'état matrimonial, une distribution de stade de vie, une distribution de type d'emploi et une distribution de type d'industrie ;

la caractéristique de déplacement d'utilisateur comprend au moins l'un parmi : un mode de déplacement, une distribution point de départ-mode de destination et une distribution point de départ-mode de déplacement-mode de destination.

2. Procédé selon la revendication 1, dans lequel le modèle initial comprend en outre un décodeur ;

le décodeur reconstruit la caractéristique de région selon la représentation de caractéristiques de la région échantillon ;

le processus d'entraînement a également un objectif consistant à minimiser une différence entre la caractéristique de région reconstruite par le décodeur et la caractéristique de région extraite de la région échantillon.

3. Procédé selon la revendication 2, dans lequel dans le processus d'entraînement du modèle initial, des paramètres du discriminateur sont optimisés à l'aide d'une première fonction de perte, des paramètres de l'encodeur sont optimisés à l'aide d'une deuxième fonction de perte, d'une troisième fonction de perte et d'une quatrième fonction de perte, des paramètres du classificateur sont optimisés à l'aide de la troisième fonction de perte, et des paramètres du décodeur sont optimisés à l'aide de la quatrième fonction de perte ;

la première fonction de perte est utilisée pour minimiser une différence entre un résultat d'identification de la région échantillon par le discriminateur et le résultat d'annotation ;

la deuxième fonction de perte est utilisée pour minimiser la différence de l'identification des régions échantillons appartenant aux districts avec différents niveaux de risque par le discriminateur ;

la troisième fonction de perte est utilisé pour minimiser la différence entre le résultat de l'identification de la région échantillon par le classificateur et le résultat d'annotation ;

la quatrième fonction de perte est utilisée pour minimiser la différence entre la caractéristique de région reconstruite par le décodeur et la caractéristique de région extraite de la région échantillon.

4. Procédé de prédiction de risque régional, **caractérisé en ce qu'**il comprend :

l'extraction (401) de caractéristiques de région d'un bloc cible ; et

l'entrée (402) des caractéristiques de région dans un modèle de prédiction de risque, et la détermination d'un niveau de risque de la région cible selon un résultat délivré en sortie par le modèle de prédiction de risque ;

dans lequel le modèle de prédiction de risque est préétabli à l'aide du procédé selon l'une quelconque des revendications 1 à 3.

5. Procédé selon la revendication 4, dans lequel le niveau de risque est un niveau de risque de propagation épidémique.

6. Appareil permettant d'établir un modèle de prédiction de risque, comprenant :

une unité d'acquisition de données (501) configurée pour acquérir (101) des données d'entraînement, les données d'entraînement comprenant un ensemble de régions échantillons et des résultats d'annotation d'un niveau de risque de chaque région échantillon dans l'ensemble de régions échantillons et un niveau de risque d'un district auquel appartient chaque région échantillon ; et

une unité d'entraînement de modèle (502) configurée pour entraîner (102) un modèle initial comprenant un encodeur, un discriminateur et un classificateur à l'aide des données d'entraînement, et obtenir le modèle de prédiction de risque à l'aide de l'encodeur et du classificateur dans le modèle initial après le processus d'entraînement ;

dans lequel l'encodeur met en oeuvre une opération de codage à l'aide de caractéristiques de région des régions échantillons pour obtenir une représentation de caractéristiques de chaque région échantillon ; le discriminateur identifie le niveau de risque du district auquel appartient la région échantillon selon la représentation de caractéristiques de la région échantillon ; le classificateur identifie le niveau de risque de la région échantillon selon la représentation de caractéristiques de la région échantillon ; le modèle initial a des objectifs d'entraînement consistant à minimiser une différence d'identification des régions échantillons appartenant aux districts avec différents niveaux de risque par le discriminateur, et à minimiser une différence entre le résultat d'identification de la région échantillon par le classificateur et le résultat d'annotation, l'appareil comprenant en outre :

une unité d'extraction de caractéristique (503) configurée pour acquérir la caractéristique de région de la région échantillon, comprenant au moins l'une parmi : une caractéristique de POI de type prédéfini environnant, une caractéristique démographique, et une caractéristique de déplacement d'utilisateur, et dans lequel la caractéristique de POI de type prédéfini environnant comprend au moins l'un parmi des informations d'une distance entre la région échantillon et un POI le plus proche d'un type prédéfini, et un degré d'exhaustivité d'installations d'habitation dans une plage de distance prédéfinie de la région échantillon ;

la caractéristique démographique comprend au moins l'une parmi : une condition de densité de population, une distribution de distance de navettage, une distribution d'âge, une distribution de sexe, une distribution de revenu, une distribution de capacité de consommation, une distribution de niveau d'éducation, une distribution d'état matrimonial, une distribution de stade de vie, une distribution de type d'emploi et une distribution de type d'industrie ;

la caractéristique de déplacement d'utilisateur comprend au moins l'un parmi : un mode de déplacement, une distribution point de départ-mode de destination et une distribution point de départ-mode de déplacement-mode de destination.

7. Appareil selon la revendication 6, dans lequel le modèle initial comprend en outre un décodeur ;

le décodeur reconstruit la caractéristique de région selon la représentation de caractéristiques de la région échantillon ;

le processus d'entraînement a également un objectif consistant à minimiser une différence entre la caractéristique de région reconstruite par le décodeur et la caractéristique de région extraite de la région échantillon.

8. Appareil selon la revendication 7, dans lequel dans le processus d'entraînement du modèle initial, l'unité d'entraînement de modèle optimise des paramètres du discriminateur à l'aide d'une première fonction de perte, optimise des paramètres de l'encodeur à l'aide d'une deuxième fonction de perte, d'une troisième fonction de perte et d'une quatrième fonction de perte, optimise des paramètres du classificateur à l'aide de la troisième fonction de perte, et optimise des paramètres du décodeur à l'aide de la quatrième fonction de perte ;

la première fonction de perte est utilisée pour minimiser une différence entre un résultat d'identification de la région échantillon par le discriminateur et le résultat d'annotation ;

la deuxième fonction de perte est utilisée pour minimiser la différence de l'identification des régions échantillons appartenant aux districts avec différents niveaux de risque par le discriminateur ;

la troisième fonction de perte est utilisé pour minimiser la différence entre le résultat de l'identification de la région échantillon par le classificateur et le résultat d'annotation ;

la quatrième fonction de perte est utilisée pour minimiser la différence entre la caractéristique de région reconstruite par le décodeur et la caractéristique de région extraite de la région échantillon.

9. Appareil de prédiction de risque régional, **caractérisé en ce qu'**il comprend :

une unité d'extraction de caractéristiques (601) configurée pour extraire des caractéristiques de région d'un bloc cible ; et

une unité de prédiction de risque (602) configurée pour entrer les caractéristiques de région dans un modèle de prédiction de risque, et déterminer un niveau de risque de la région cible selon un résultat délivré en sortie par le modèle de prédiction de risque ;

dans lequel le modèle de prédiction de risque est préétabli par l'appareil selon l'une quelconque des revendications 6 à 8.

10. Appareil selon la revendication 9, dans lequel le niveau de risque est un niveau de risque de propagation épidémique.

11. Dispositif électronique comprenant :

au moins un processeur ; et

une mémoire connectée à l'au moins un processeur par communications ;

dans lequel la mémoire stocke des instructions exécutables par l'au moins un processeur pour amener l'au moins un processeur à mettre en oeuvre le procédé selon l'une quelconque des revendications 1 à 5.

12. Support de stockage non transitoire lisible par ordinateur comprenant des instructions d'ordinateur, qui, lorsqu'elles sont exécutées par un ordinateur, amènent l'ordinateur à mettre en oeuvre le procédé selon l'une quelconque des revendications 1 à 5.

**13.** Produit programme d'ordinateur comprenant un programme d'ordinateur qui, lorsqu'il est exécuté par un processeur, amène le processeur à mettre en oeuvre le procédé selon l'une quelconque des revendications 1 à 5.

101

Acquiring training data, the training data including a sample region set and annotation results of a risk grade of each sample region in the sample region set and a risk grade of a district to which each sample region belongs

102

Training an initial model including an encoder, a discriminator and a classifier using the training data, and obtaining the risk prediction model using the encoder and the classifier in the initial model after the training process

**Fig. 1**

**Fig. 2**

$n_r^T$

$n_h^T$

$n_t^T$

Concatenate

$n^T$

Encoder

$\tilde{n}^T$

Classifier

Risk grade

**Fig. 3**

---

401

Extracting region features of a target block

402

Inputting the region features into a risk prediction model, and determining a risk grade of the target region according to a result output by the risk prediction model

**Fig. 4**

500

501

Data acquiring unit

503

Feature extracting unit

502

Model training unit ⟷ Initial model

⇩

Risk prediction model

**Fig. 5**

600

601

Feature extracting unit

602

Risk predicting unit ⟷ Risk prediction model

**Fig. 6**

700

701                        702                        703

| Computing unit |        | ROM |        | RAM |

704

705

| I/O interface |

706          707          708          709

| Input unit |   | Output unit |   | Storage unit |   | Communication unit |

**Fig. 7**

**EP 4 040 353 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2020115159533
- CN 109902880 A
- CN 111626490 A
- CN 110689184 A